# EUROPEAN PATENT APPLICATION

(11) **EP 1 314 422 A2**
(43) Date of publication of application: **28.05.2003**
(21) Application number: 02016077.6
(22) Date of filing: 19.07.2002
(51) Int. Cl.: A61K 9/00, A61K 9/14, A61K 9/20

(54) **Orally disintegrating solid preparations and processes for the production thereof**

(30) Priority: 14.11.2001 JP 2001349063
(71) Applicant: SCG, INC., Hiki-gun, Saitama 355-0155 (JP)
(72) Inventor: Katsuta, Toshifumi, Sakado-shi, Saitama 350-0241 (JP)
(74) Representative: Helbing, Jörg, Dr. Dipl.-Chem.

(57) **Abstract**

An object of the present invention is to develop a pharmaceutical preparation which is rapidly dissolved or disintegrated in an oral cavity and a process for the formulation thereof. The orally disintegrating solid pharmaceutical preparations are obtained by dispersively ejecting melts containing sugars (e.g., monosaccharides, disaccharides, sugar alcohols, etc.) and pharmaceutically active components under an atmosphere at a temperature equal to or lower than the solidifying point of the sugar, solidifying in a filamentous or flocculent state, and subsequently collecting flocculent masses followed by molding into a given dosage form and processes for the production thereof are also provided. The orally disintegrating solid preparations are safe, sweet and easily ingestible which can be rapidly dissolved or disintegrated in the oral cavity since they have a high porosity due to the formulation from filamentous or flocculent masses and contain highly water-soluble sugars as their major bases.

## Description

### FIELD OF THE INVENTION

The present invention relates to a solid pharmaceutical preparation which is rapidly disintegrated or dissolved in an oral cavity. More particularly, the invention relates to an orally disintegrating solid pharmaceutical preparation wherein a flocculent product obtained from a melt containing sugar and a pharmaceutically active component is formulated into a desired dosage form, which is rapidly disintegrated or dissolved in an oral cavity.

### BACKGROUND OF THE INVENTION

Recently, populations of the aged have rapidly increased, and drug preparations for the elderly have gained attention. In respect to the issue of what is optimal preparation for the elderly, a report entitled "Study on optimal production of novel preparations and novel package containers for administering to the elderly" (Masayasu Sugihara, et al., Tokyo Women's Medical University; "Yakuji Nippo", published on Aug. 2, 1989, The Yakuji Nippo Limited., Japan) was prepared as a follow up to the project for Silver Science Study by the Ministry of Health and Welfare, Japan. In that report, agents brought suitable preparation for the elderly included (1) orally soluble preparations, (2) paste preparations, and (3) jelly preparations. And in that same paper, the orally soluble solid preparation for the elderly was introduced, wherein a sweetening component is added, in view of easy ingestion, to an oil base selected as a base capable of being dissolved at an oral cavity temperature to afford a mixture and the resultant mixture is then heat-melted to give a melt which is packed into PTP and allowed to stand for cooling.

There is also a report entitled "Preparations ingestable for patients afflicted with dysphagia without the need for water" in "Risu Fakkusu", Vol. 1743 (published June 28, 1995, K.K. Yakugyokai Shinbun, Japan) wherein the preparation readily disintegrated in the oral cavity has been desirable to satisfy the needs of elderly patients afflicted with dysphagia who complain that it is difficult to ingest tablets and capsules.

In order to correspond to such needs from medical care facilities, recently, various types of solid preparations with increased solubility or disintegrability have been vigorously developed as described herein below.

For example, JP, B2, 58-24410 (1983) discloses a tablet of which the water-soluble rate is from 10 to 25 seconds, obtained by mixing ingredients for the tablet with a solvent which will be frozen at -30 to +20°C, granulating this mixture in liquid nitrogen, and tabletting the frozen granules in a tabletting machine previously cooled to from -20 to -25°C with a pressure of 3000 kp/cm². A flexible tablet has been proposed in JP, B2, 60-30496 (1985), and the tablet is directed for confections or medicines which are capable of being chewed, obtained by directly compressing and molding a dry blend containing xylitol and a polyalcohol such as sorbitol, mannitol and the like.

JP, A, 60-4124 (1985) discloses a tablet obtained by mixing microcrystalline cellulose, starch and bibasic calcium phosphate with a low soluble drug (such as metolazone and triamterene) which has been finely pulverized to a particle size of less than about 155µm to form a dry mixture, followed by compression and molding. The tablet has demonstrated experimental results wherein disintegration occurs in water within 10 to 22 seconds, and the solubility of the low soluble agent (in 0.1N hydrochloric acid solution) is enhanced. Moreover, JP, A, 5-271054 (1993) discloses a tablet having an appropriate disintegrability and solubility in the oral cavity, which is obtained by admixing the pharmaceutically active component and sugars with water at an extent to wet the particle surfaces of the sugars to form a mixture, followed by tabletting. According to the test results, it has been described that the tablet has a dissolution time period from 0.3 to 1.5 min and a disintegration time period from 0.2 to 0.9 min in the oral cavity.

For the above preparations, it appears that in each case a certain measure of results has been obtained in light of the dissolution and disintegration rates and the preparations are capable of responding to the needs of the medical fields. However, in light of the production processes, methods which are not necessarily suitable for practical applications, such as the use of liquid nitrogen, have been applied. There are dry methods and wet methods in formulation of orally disintegrating solid preparations. The wet methods are further classified depending on the difference in removal methods of solvents, i.e., into air drying, vacuum drying, and freeze drying, etc. Methods applied for the formulation of general solid preparations have also been employed, wherein each method has respective characteristics.

For example, in the drying method, the number of steps may be reduced since no solvent is used, but only dry particles lead to weak binding powers between particles. In order to enhance the binding power, tabletting pressure must be increased, and as the result, porosity becomes small leading to an effect of making the tablet disintegration rate slow.

In the freeze drying method, since solvents are removed in a frozen state under a vacuum, void openings are readily formed during formulation compared to the drying method and vacuum drying method, so that it would be easy to increase the specific surface area and to penetrate solvents into the void openings. Therefore, it has the advantage of producible technique for rapidly disintegrable preparations. In the freeze drying method; however, there are drawbacks in that the tablet strength is insufficient, continuous drying is difficult and production efficiency is inferior due to the dependency on batch modes. Therefore, it may be said that the freeze drying method is applied mainly for drying of powder preparations and has less application in drying solid preparations such as tablets.

As mentioned above, for rapidly soluble or disintegrable solid preparations, it is difficult to obtain the preparation with properties as expected even when ingenious plans are made for either the additives or in the formulation methods. Thus, it can be said that it is essential to study from both sides of the selection of additives and devising of formulation methods suitable for the composition, based on significant understanding of the material properties of drugs.

### SUMMARY OF THE INVENTION

Under the aforementioned technical background, an object of the present invention is to develop a solid pharmaceutical preparation with not only a rapid dissolution or disintegration rate but also a property of retaining a required strength. Another object of the present invention is to develop a process for the formulation enabling industrial utilization of such a solid preparation more efficiently.

The present inventors have performed an intensive study to achieve the above objects. As a result, the present inventors have obtained the following findings:
(a) solid preparations can be prepared by steps
   (i) dispersively ejecting a melt containing a sugar and the pharmaceutically active component into the atmosphere at a temperature equal to or lower than the solidifying point of the sugar to form a flocculent product (fleecy mass), and
   (ii) subjecting the flocculent product to formulation; and
(b) such solid preparations possess not only dissolution and disintegration rates in the oral cavity, equivalent to or faster than those of conventional similar preparations, but also strength properties against dropping, of securing the preparations against breakage in the shipping and distribution processes.

The present invention has been completed based on such findings.

The present invention provides:
(1) an orally disintegrating solid preparation which is prepared by the steps:
   dispersively ejecting a melt containing a sugar and one or more pharmaceutically active components into the atmosphere at a temperature equal to or lower than the solidifying point of the sugar,
   cooling and solidifying the melt to form a flocculent product, and
   then formulating the flocculent product into a desired dosage form;
(2) the orally disintegrating solid preparation according to the above (1), wherein the pharmaceutically active component is selected from the group consisting of drugs for the central nervous system, drugs for cardiovascular organs, drugs for respiratory organs, drugs for gastrointestinal organs, hormone drugs, vitamins, drugs for tumors, drugs for allergy and narcotics; and
(3) a process for producing an orally disintegrating solid preparation, which comprises
   (i) adding one or more pharmaceutically active components to a sugar, if necessary, in admixture with an additive, to form a powder mixture,
   (ii) heat-melting the powder mixture to give a liquid melt,
   (iii) dispersively ejecting the liquid melt from pores or nozzles under the atmosphere at a temperature equal to or lower than the solidifying point of the sugar,
   (iv) cooling and solidifying the melt to form a flocculent product, and
   (v) then formulating the flocculent product into a desired dosage form.

The above objectives and other objectives, features, advantages, and aspects of the present invention are readily apparent to those skilled in the art from the following disclosures. It should be understood, however, that the description of the specification including the following detailed description of the invention, examples, etc. is illustrating preferred embodiments of the present invention and given only for explanation thereof. It will become apparent to the skilled in the art that a great number of variations and/or alterations (or modifications) of this invention may be made based on knowledge from the disclosure in the following parts and other parts of the specification without departing from the spirit and scope thereof as disclosed herein. All of the patent publications and reference documents cited herein for illustrative purposes are hereby incorporated by reference into the present disclosure.

### DETAILED DESCRIPTION OF THE INVENTION

The sugar as used for the orally disintegrating solid pharmaceutical preparations of the present invention refers to monosaccharides, disaccharides and sugar alcohols. The sugars as used herein include one or more compounds selected from the group consisting of such monosaccharides, disaccharides and sugar alcohols. Suitable sugars are compounds of which the melting point ranges from 70 to 250°C Preferably, more suitable sugars are compounds of which the melting point ranges from 80 to 220°C, in light of thermal stability, melting and solidifying properties. Examples of such sugars having a melting point of from 80 to 220°C are fructose, glucose, sucrose, lactose, maltose, lactulose, erythritol, xylitol, mannitol, sorbitol, maltitol, etc. Preferable sugars are sucrose, maltose, lactulose, erythritol, xylitol, sorbitol, etc. More preferable sugars are, in terms of the solidifying property and ease in handling, maltose, mixtures of sucrose and maltose, and the like.

The pharmaceutically active components which can be used for the orally disintegrating solid preparations of the present invention are not especially limited so long as they are drugs or agents which are neither vaporized nor thermally degraded, etc. at the melting temperature of the sugar used as a base material. The pharmaceutically active components may be widely selected and applicable irrespective of their pharmacological properties. For instance, drugs for the central nervous system, drugs for cardiovascular organs, drugs for respiratory organs, drugs for gastrointestinal organs, hormone drugs, vitamins, drugs for tumors, drugs for allergy and narcotics, etc. are applicable. Specifically representatives of such drugs include the following:
1) Drugs for the central nervous system include hypnotics, sedatives and anxiolytics such as estazolam, triazolam, nitrazepam, diazepam, and chlordiazepoxide; analgesic-antipyretic and antiinflammatory drugs such as indomethacin, sodium diclofenac, tiaramide hydrochloride, ibuprofen, ketoprofen, naproxen, flurbiprofen, and sodium loxoprofen; and psychotherapeutic and neurotherapeutic drugs such as chlorpromazine hydrochloride, etizolam, amitriptyline hydrochloride, haloperidol, and tiapride hydrochloride;
2) Drugs for cardiovascular organs include cardiotonic drugs such as etilefrin hydrochloride, and proscillaridin; antiarrhythmic drugs such as atenolol, alprenolol hydrochloride, carteolol hydrochloride, propranolol hydrochloride, pindolol, and mexiletine hydrochloride; antihypertensive drugs such as todralazine hydrochloride, hydralazine hydrochloride, rescinnamine, reserpine, nicardipine hydrochloride, prazosin hydrochloride, and metoprolol tartrate; vasodilator drugs such as diltiazem hydrochloride, etafenone hydrochloride, trimetazidine hydrochloride, dipyridamole, and nifedipine; and others including cinnarizine, vinpocetine, dihydroergotoxine mesylate, etc.;
3) Drugs for respiratory organs include antitussives such as ephedrine hydrochloride; expectorant drugs such as bromhexine hydrochloride, and eprazinone hydrochloride; and bronchodilators such as fenoterol hydrobromide, and salbutamol sulfate;
4) Drugs for gastrointestinal organs include antidiarrheal drugs and gastrointestinal tract-normalizing drugs such as loperamide hydrochloride; drugs for digestive ulcers such as pyrenezepine hydrochloride; laxatives such as sodium picosulfate; and others including domperidone, metoclopramide and the like;
5) Hormone drugs include adrenal hormone agents such as dexamethasone, betamethasone, and prednisolone; and luteinizing hormone agents such as chlormadinone hydrochloride;
6) Vitamins include vitamin D drugs such as alpha-calcidol, and calcitriol; vitamin B drugs such as thiamine hydrochloride, thiamine disulfide, riboflavin, nicotinic acid, pyridoxine hydrochloride, pyridoxal phosphate, cobamamide, and mecobalamin; etc.;
7) Drugs for tumors include antimetabolites such as fluorouracil;
8) Drugs for allergy include antihistaminics such as diphenhydramine hydrochloride, mequitazine, cyproheptadine hydrochloride, and chlorpheniramine maleate; and others including azelastine hydrochloride, ketotifen fumarate and the like; and
9) Narcotic agents include morphine hydrochloride, codeine phosphate and the like.

For the pharmaceutically active components used in the present invention, especially preferable agents include drugs classified into psychotherapeutic and neurotherapeutic agents, antiarrhythmics, vasodilators, and antidiarrheal agents.

The amount of the pharmaceutically active component in the mixture wherein the pharmaceutically active component is admixed with sugars may vary mainly depending on the dosage of the drug. However, the pharmaceutically active component is admixed usually at from approximately 0.0001 to 70% by weight, preferably at from approximately 0.0001 to 30% by weight, and more preferably at from approximately 0.0001 to 10% by weight. For example, in a case when alpha-calcidol is used at 0.25 µ g/tablet as the pharmaceutically active component for one dosage, it is admixed at approximately 0.0001% by weight; in a case when procaterol hydrochloride is employed at 25 µ g/tablet for one dosage, at approximately 0.01% by weight; in a case when ketotifen fumarate, etizolam or others at 1 mg/tablet for one dosage, at approximately 0.5% by weight; in a case when nifedipine or others at 20 mg/tablet for one dosage, at approximately 10% by weight; in a case when flurbiprofen or others at 40 mg/tablet for one dosage, at approximately 20% by weight; and in a case when ibuprofen or others at 100 mg/tablet for one dosage, at approximately 50% by weight.

Conventionally utilizable additives may be added to the orally disintegrating solid preparations according to the present invention so long as they do not adversely affect efficacies and formulations. Examples of such additives are disintegrants, binders, souring agents, artificial sweeteners, flavoring agents, lubricants, coloring agents and the like.

Specifically, the disintegrant includes starch, the binder does gelatin, the souring agent does citric acid, tartaric acid and malic acid, the artificial sweetener does sodium saccharin, aspartame and stevia, the flavoring agent does lemon, orange and menthol, the lubricant does sucrose fatty acid ester and talc, and the coloring agent does edible yellow No. 5, edible red No.2 and edible blue No.2. These additives may be added either during the mixing of the sugar and the pharmaceutically active component or during the formulation of the flocculent product.

The orally disintegrating solid preparations of the present invention can be prepared by novel processes:
(a) forming a flocculent product (fleecy mass) from a molten powder mixture of the sugar and the pharmaceutically active component, and
(b) directly formulating the flocculent product into a desired form or shape without using any solvent, etc.

First, pharmaceutically active components are mixed with sugars, if necessary, in admixture with one or more additives as aforementioned, and all the ingredients are well blended. The mixture is supplied in small portions to a melting furnace, ordinarily heated to from approximately 170 to approximately 250°C in a flocculent mass producing machine (Tsukuda Original, Japan) and thermally melted to form hot melts. Next, the resultant melt is dispersively ejected portionwise in a filamentous or flocculent state into an atmosphere at a temperature equal to or lower than the solidifying point of the sugars used, usually at an ambient temperature or less, and cooled and solidified to yield flocculent masses containing the pharmaceutically active components. Upon the melting and solidifying processes for unstable drugs which are easily oxidized, it is better to carry out such processes under an inert gas atmosphere.

The flocculent products containing the above pharmaceutically active components can be weighed at an amount required for pharmaceutical preparations depending on their size or content level and molded into dosage forms with a predetermined size, with a cylinder type compression tabletting machine via adjusting a pushing position of a plunger to vary loads thereon.

For example, when molded at approximately 200 mg into tablets with a size of 1 cm³ (volume), the flocculent products are required to be compressed with a load of approximately 500 g; when a tablet size of 0.5 cm³ is required, the load needs approximately 3 kg; and when a tablet size of 0.3 cm³ is required, the load needs approximately 15 kg.

The solid preparations thus obtained herein are rapidly dissolved or disintegrated since water is readily penetrated into numerous pores and cavities existing in the preparations due to three dimensional network structure of the flocculent product (fleecy mass), plus the sugars used as the bases are greatly water-soluble.

In addition, the preparations with a three dimensional network structure are highly resistant against an impact by dropping. For example, the preparations have a strength almost free of being broken or fractured even when dropped from 230 cm in height.

### EXAMPLES

Described below are examples of the present invention which are provided only for illustrative purposes, yet these are not to limit the scope of the present invention. All the examples were or can be practiced using standard techniques well or conventionally known to those of ordinary skill in the art unless otherwise specified.

### Example 1

Powdered nifedipine (2 g) was added to 40 g of sucrose powders, and the mixture was well blended. Thereafter, the mixture was placed in a thin stainless steel melting vessel, and stirred thoroughly with heating on an oil bath at 200°C to afford a homogeneous orange melt mixture. After this melt mixture was placed together with the melting vessel in a desiccator and cooled down to room temperature, it was taken out from the melting vessel to yield 38.8 g of an orange melt mixture. This melt mixture was ground in a mortar to yield 35.4 g of powders. The powders were placed in a hopper of a flocculent mass-producing machine, supplied portionwise from a hopper into a melting furnace in a vibrating manner, ejected fleecily or filamentously at a burst in the form of a very hot liquid into a space for solidification in a flocculating machine at room temperature while melting in the melting furnace heated from approximately 170 to 250°C with an electric heater, and rapidly solidified with cooling down to room temperature to produce flocculent masses. The flocculent masses were collected by a collecting machine to yield 24.5 g of pale yellowish orange flocculent masses. The resultant flocculent masses were divided into aliquots of approximately 200 mg, and then compressed with a cylinder type compression tabletting machine to form solid preparations wherein adjustment of a pushing position of a plunger (or punch) to get a volume of 1 cm³ and compression with a load of approximately 500 g led to the production of 60 tablets (each tablet size: 12.5 mm diameter and approximately 8 mm thickness). Similarly, tabletting using adjustment of the plunger pushing position to get a volume of 0.5 cm³ and compression with a load of approximately 3 kg led to the production of solid preparations, 54 tablets (each tablet size: 12.5 mm diameter and approximately 4 mm thickness).

### Example 2

Powdered nifedipine (2 g) was added to 40 g of maltose powders and the mixture was well blended, then placed in a thin stainless steel melting vessel, and stirred thoroughly on an oil bath at 165°C to afford a homogeneous yellow melt mixture. After this melt mixture was placed together with the melting vessel in a desiccator and cooled down to room temperature, it was removed from the vessel to yield 38.9 g of a yellow melt mixture. This melt mixture was ground in a mortar to yield 38.4 g of pale yellow powders. The powders were placed in a hopper of a flocculent mass-producing machine, and supplied portionwise from the hopper to a melting furnace in a vibrating manner, ejected fleecily or filamentously at a burst in the form of a very hot liquid into a space for solidification in a flocculating machine at room temperature while melting in the melting furnace heated to from approximately 170 to 250°C with an electric heater, and rapidly solidified with cooling down to room temperature to produce flocculent masses. The flocculent masses were collected by a collecting machine to yield 28.8 g of pale yellow flocculent masses. The resultant flocculent masses were divided into aliquots of approximately 200 mg, and then compressed with a cylinder type compression tabletting machine to form solid preparations wherein adjustment of a pushing position of a plunger to get a volume of 1 cm³ and compression with a load of approximately 400 g led to the production of 60 tablets (each tablet size: 12.5 mm diameter and approximately 8 mm thickness). Similarly, tabletting using adjustment of the plunger pushing position to get a volume of 0.5 cm³ and compression with a load of 2 kg led to the production of solid preparations, 60 tablets (each tablet size: 12.5 mm diameter and approximately 4 mm thickness). Further, tabletting using adjustment of the plunger pushing position to get a volume of 0.3 cm³ and compression with a load of 15 kg led to the production of solid preparations, 21 tablets (each tablet size: 12.5 mm diameter and approximately 2.5 mm thickness).

### Example 3

Powdered nifedipine (2 g) was added to 40 g of sucrose powders and the mixture was well blended, then placed in a hopper of a flocculent mass-producing machine, supplied in small portions from the hopper to a melting furnace in a vibrating manner, ejected fleecily or filamentously at a burst in the form of a very hot liquid into a space for solidification in a flocculating machine at room temperature while melting in the melting furnace heated from approximately 170 to 250°C with an electric heater, and rapidly solidified with cooling down to room temperature to produce flocculent masses. The flocculent masses were collected by a collecting machine to yield 28.2 g of pale yellow flocculent masses. The resultant flocculent masses were divided into aliquots of approximately 200 mg, and then compressed with a cylinder type compression tabletting machine to form solid preparations wherein adjustment of a pushing position of a plunger to get a volume of 1 cm³ and compression with a load of approximately 500 g led to the production of 60 tablets (each tablet size: 12.5 mm diameter and approximately 8 mm thickness). Similarly, tabletting using adjustment of the plunger pushing position to get a volume of 0.5 cm³ and compression with a load of approximately 3 kg led to the production of solid preparations, 50 tablets (each tablet size: 12.5 mm diameter and approximately 4 mm thickness). Further, tabletting using adjustment of the plunger pushing position to get a volume of 0.3 cm³ and compression with a load of approximately 15 kg led to the production of solid preparations, 24 tablets (each tablet size: 12.5 mm diameter and approximately 2.5 mm thickness).

### Example 4

Powdered nifedipine (2 g) was added to 40 g of maltose powders, and the mixture was well blended using the same procedure as in Example 3 to yield 29.7 g of pale yellow flocculent masses. The resultant flocculent masses were divided into aliquots of approximately 200 mg, and then compressed to afford solid preparations; i.e., compression with a load of approximately 400 g into 60 tablets with a volume of 1 cm³ per tablet, compression with a load of approximately 2 kg into 50 tablets with a volume of 0.5 cm³ per tablet, and compression with a load of approximately 16 kg into 30 tablets with a volume of 0.3 cm³ per tablet.

### Example 5

Maltose powders (8 g) and nifedipine powders (2 g) were added to 32 g of sucrose powders, and the mixture was well blended using the same procedure as in Example 3 to yield 32.5 g of pale yellow flocculent masses.
The resultant flocculent masses were divided into aliquots of approximately 200 mg, and then compressed to afford solid preparations; i.e., compression with a load of approximately 500 g into 60 tablets with a volume of 1 cm³ per tablet, compression with a load of approximately 3 kg into 60 tablets with a volume of 0.5 cm³ per tablet, and compression with a load of approximately 14 kg into 35 tablets with a volume of 0.3 cm³ per tablet.

### Example 6

Maltose powders (20 g) and nifedipine powders (2 g) were added to 20 g of sucrose powders, and the mixture was well blended using the same procedure as in Example 3 to yield 33.4 g of pale yellow flocculent masses. The resultant flocculent masses were divided into aliquots of approximately 200 mg, and then compressed to afford solid preparations; i.e., compression with a load of approximately 500 g into 60 tablets with a volume of 1 cm³ per tablet, compression with a load of approximately 3 kg into 60 tablets with a volume of 0.5 cm³ per tablet, and compression with a load of approximately 15 kg into 40 tablets with a volume of 0.3 cm³ per tablet.

### Example 7

Maltose powders (32 g) and nifedipine powders (2 g) were added to 8 g of sucrose powders, and the mixture was well blended using the same procedure as in Example 3 to yield 31.5 g of pale yellow flocculent masses. The resultant flocculent masses were divided into aliquots of approximately 200 mg, and then compressed to afford solid preparations; i.e., compression with a load of approximately 400 g into 60 tablets with a volume of 1 cm³ per tablet, compression with a load of approximately 2 kg into 50 tablets with a volume of 0.5 cm³ per tablet, and compression with a load of approximately 17 kg into 34 tablets with a volume of 0.3 cm³ per tablet.

### Example 8

Lactulose powders (8 g) and nifedipine powders (2 g) were added to 32 g of sucrose powders, and the mixture was well blended using the same procedure as in Example 3 to yield 25.8 g of pale yellow flocculent masses. The resultant flocculent masses were divided into aliquots of approximately 200 mg, and then compressed to afford solid preparations; i.e., compression with a load of approximately 500 g into 60 tablets with a volume of 1 cm³ per tablet, compression with a load of approximately 3 kg into 40 tablets with a volume of 0.5 cm³ per tablet, and compression with a load of approximately 14 kg into 24 tablets with a volume of 0.3 cm³ per tablet.

### Example 9

Maltose powders (20 g) and domperidone powders (2 g) were added to 20 g of sucrose powders, and the mixture was well blended using the same procedure as in Example 3 to yield 27.0 g of white flocculent masses. The resultant flocculent masses were divided into aliquots of approximately 200 mg, and then compressed to afford solid preparations; i.e., compression with a load of approximately 500 g into 60 tablets with a volume of 1 cm³ per tablet, compression with a load of approximately 3 kg into 40 tablets with a volume of 0.5 cm³ per tablet, and compression with a load of approximately 15 kg into 30 tablets with a volume of 0.3 cm³ per tablet.

### Example 10

Maltose powders (20 g) and pindolol powders (1 g) were added to 20 g of sucrose powders, and the mixture was well blended using the same procedure as in Example 3 to yield 34.4 g of white to pale yellowish brown flocculent masses.

The resultant flocculent masses were divided into aliquots of approximately 200 mg, and then compressed to afford solid preparations; i.e., compression with a load of approximately 500 g into 60 tablets with a volume of 1 cm³ per tablet, compression with a load of approximately 3 kg into 60 tablets with a volume of 0.5 cm³ per tablet, and compression with a load of approximately 14 kg into 45 tablets with a volume of 0.3 cm³ per tablet.

### Example 11

Maltose powders (20 g) and etizolam powders (0.2 g) were added to 20 g of sucrose powders, and the mixture was well blended using the same procedure as in Example 3 to yield 35.3 g of white flocculent masses. The resultant flocculent masses were divided into aliquots of approximately 200 mg, then compressed to afford solid preparations; i.e., compression with a load of approximately 500 g into 60 tablets with a volume of 1 cm³ per tablet, compression with a load of approximately 3 kg into 60 tablets with a volume of 0.5 cm³ per tablet, and compression with a load of approximately 14 kg into 49 tablets with a volume of 0.3 cm³ per tablet.

### Example 12

Maltose powders (20 g) and ketotifen fumarate powders (0.2 g) were added to 20 g of sucrose powders, and the mixture was well blended using the same procedure as in Example 3 to yield 28.3 g of white flocculent masses. The resultant flocculent masses were divided into aliquots of approximately 200 mg, and then compressed to afford solid preparations; i.e., compression with a load of approximately 500 g into 60 tablets with a volume of 1 cm³ per tablet, compression with a load of approximately 3 kg into 40 tablets with a volume of 0.5 cm³ per tablet, and compression with a load of approximately 15 kg into 35 tablets with a volume of 0.3 cm³ per tablet.

### Example 13

Maltose powders (20 g) and vinpocetine powders (1 g) were added to 20 g of sucrose powders and the mixture was well blended using the same procedure as in Example 3 to yield 25.7 g of white flocculent masses. The resultant flocculent masses were divided into aliquots of approximately 200 mg, and then compressed to afford solid preparations; i.e., compression with a load of approximately 500 g into 50 tablets with a volume of 1 cm³ per tablet, compression with a load of approximately 3 kg into 40 tablets with a volume of 0.5 cm³ per tablet, and compression with a load of approximately 14 kg into 32 tablets with a volume of 0.3 cm³ per tablet.

### Example 14

Maltose powders (20 g) and mequitazine powders (0.6 g) were added to 20 g of sucrose powders and the mixture was well blended using the same procedure as in Example 3 to yield 21.9 g of white to pale yellowish brown flocculent masses. The resultant flocculent masses were divided into aliquots of approximately 200 mg, and then compressed to afford solid preparations; i.e., compression with a load of approximately 500 g into 40 tablets with a volume of 1 cm³ per tablet, compression with a load of approximately 3 kg into 35 tablets with a volume of 0.5 cm³ per tablet, and compression with a load of approximately 14 kg into 27 tablets with a volume of 0.3 cm³ per tablet.

### Example 15

Maltose powders (20 g) and loperamide hydrochloride powders (0.2 g) were added to 20 g of sucrose powders and the mixture was well blended using the same procedure as in Example 3 to yield 29.6 g of white flocculent masses.
The resultant flocculent masses were divided into aliquots of approximately 200 mg, and then compressed to afford solid preparations; i.e., compression with a load of approximately 500 g into 60 tablets with a volume of 1 cm³ per tablet, compression with a load of approximately 3 kg into 50 tablets with a volume of 0.5 cm³ per tablet, and compression with a load of approximately 15 kg into 30 tablets with a volume of 0.3 cm³ per tablet.

### Example 16

Nifedipine powders (1 g) were added to 20 g of sucrose powders, and the mixture was well blended using the same procedure as in Example 3 to yield 20.3 g of pale yellow flocculent masses. The resultant flocculent masses were divided into aliquots of approximately 200 mg, and then compressed to afford solid preparations; i.e., compression with a load of approximately 300 g into 30 tablets with a volume of 1 cm³ per tablet, compression with a load of approximately 2 kg into 30 tablets with a volume of 0.5 cm³ per tablet, and compression with a load of approximately 15 kg into 32 tablets with a volume of 0.3 cm³ per tablet.

### Example 17

Nifedipine powders (2 g) were added to 20 g of sucrose powders, and the mixture was well blended using the same procedure as in Example 3 to yield 18.1 g of pale yellow flocculent masses. The resultant flocculent masses were divided into aliquots of approximately 200 mg, and then compressed to afford solid preparations; i.e., compression with a load of approximately 500 g into 30 tablets with a volume of 1 cm³ per tablet, compression with a load of approximately 3 kg into 30 tablets with a volume of 0.5 cm³ per tablet, and compression with a load of approximately 15 kg into 22 tablets with a volume of 0.3 cm³ per tablet.

### Example 18

Nifedipine powders (1 g) were added to 20 g of maltose powders, and the mixture was well blended using the same procedure as in Example 3 to yield 19.0 g of pale yellow flocculent masses. The resultant flocculent masses were divided into aliquots of approximately 200 mg, and then compressed to afford solid preparations; i.e., compression with a load of approximately 300 g into 30 tablets with a volume of 1 cm³ per tablet, compression with a load of approximately 2 kg into 30 tablets with a volume of 0.5 cm³ per tablet, and compression with a load of approximately 15 kg into 25 tablets with a volume of 0.3 cm³ per tablet.

### Example 19

Nifedipine powder (2 g) were added to 20 g of maltose powders, and the mixture was well blended using the same procedure as in Example 3 to yield 17.5 g of pale yellow flocculent masses. The resultant flocculent masses were divided into aliquots of approximately 200 mg, and then compressed to afford solid preparations; i.e., compression with a load of approximately 400 g into 30 tablets with a volume of 1 cm³ per tablet, compression with a load of approximately 2 kg into 25 tablets with a volume of 0.5 cm³ per tablet, and compression with a load of approximately 17 kg into 22 tablets with a volume of 0.3 cm³ per tablet.

### Example 20

Nifedipine powders (1 g) were added to 10 g of sucrose powders and 10 g of maltose powders, and the mixture was well blended using the same procedure as in Example 3 to yield 17.5 g of pale yellow flocculent masses.
The resultant flocculent masses were divided into aliquots of approximately 200 mg, and then compressed to afford solid preparations; i.e., compression with a load of approximately 500 g into 30 tablets with a volume of 1 cm³ per tablet, compression with a load of approximately 3 kg into 25 tablets with a volume of 0.5 cm³ per tablet, and compression with a load of approximately 14 kg into 25 tablets with a volume of 0.3 cm³ per tablet.

### Example 21

Nifedipine powders (2 g) were added to 10 g of sucrose powders and 10 g of maltose powders, and the mixture was well blended using the same procedure as in Example 3 to yield 17.4 g of pale yellow flocculent masses.
The resultant flocculent masses were divided into aliquots of approximately 200 mg, and then compressed to afford solid preparations; i.e., compression with a load of approximately 500 g into 30 tablets with a volume of 1 cm³ per tablet, compression with a load of approximately 4 kg into 25 tablets with a volume of 0.5 cm³ per tablet, and compression with a load of approximately 15 kg into 25 tablets with a volume of 0.3 cm³ per tablet.

### Disintegration tests, and miscellaneous assays

Disintegration tests, strength tests and calculations of density were carried out for the tablets obtained in the above examples by the following methods:

### 1) Time periods required for disintegration in water

Warm water (5 L) at approximately 37°C was placed in a 5 L beaker (water depth is approximately 21 cm), and left static. Into the static water was calmly one of tablets dropped, and the period of time required for complete deformation of each test tablet was measured as its disintegration time. The test was repeated 6 times. The minimum, maximum and mean values were recorded.

### 2) Disintegration time period in the oral cavity

The period of time required for complete deformation of the tablet with oral saliva of a healthy adult male was measured as the disintegration time. The test was repeated 3 times. The minimum, maximum and mean values were recorded.

### 3) Disintegration test with water droplets

One of tablets was plated on a flat metal plate, 4 droplets of warmed water at 37°C (approximately 0.2 ml) were dripped, and the period of time required for complete deformation of each test tablet was measured as its disintegration time. The test was repeated 6 times. The minimum, maximum and mean values were recorded.

### 4) Dropping strength

A breakage rate was measured when each test tablet was dropped from a height of 230 cm onto a concrete floor. The test was carried out using 5 tablets, and each breakage rate was calculated from the number of fractured or broken tablets.

### 5) Density

A tablet density was calculated from a volume (diameter and thickness) and weight of each test tablet.

The above test results are shown in Table 1 for Examples 1 through 15. The test results are shown in Table 2 for Examples 16 through 21 as compared with the results of the reference examples (D₁₀, D₂₀ and G).

**Table 1**

| Example No. | Tablet Size cm³/0.2g | Tablet Density g/cm³ | Disintegration Time in Water (sec.) n=6 | | | Disintegration Time in Oral Cavity (sec.) n=3 | | | Dropping Strength n=5 (Breakage Rate) |
|---|---|---|---|---|---|---|---|---|---|
| | | | Min. | Max. | Mean | Min. | Max. | Mean | |
| 1 | 1 | 0.2 | 1 | 4 | 2.2 | 7 | 10 | 8.7 | 0/5 |
| | 0.5 | 0.4 | 5 | 22 | 9.5 | 8 | 11 | 9.7 | 0/5 |
| 2 | 1 | 0.2 | 1 | 1 | 1 | 1 | 3 | 1.7 | 0/5 |
| | 0.5 | 0.4 | 1 | 1 | 1 | 1 | 2 | 1.3 | 0/5 |
| | 0.3 | 0.7 | 2 | 3 | 2.3 | ― | ― | ― | 0/5 |
| 3 | 1 | 0.2 | 2 | 5 | 3 | 4 | 5 | 4.3 | 0/5 |
| | 0.5 | 0.4 | 8 | 32 | 18.7 | 5 | 9 | 6.7 | 0/5 |
| | 0.3 | 0.7 | 19 | 41 | 29.3 | ― | ― | ― | 0/5 |
| 4 | 1 | 0.2 | 1 | 1 | 1 | 1 | 1 | 1 | 0/5 |
| | 0.5 | 0.4 | 1 | 1 | 1 | 2 | 3 | 2.7 | 0/5 |
| | 0.3 | 0.7 | 2 | 3 | 2.5 | ― | ― | ― | 0/5 |
| 5 | 1 | 0.2 | 4 | 21 | 8 | 2 | 5 | 3.3 | 0/5 |
| | 0.5 | 0.4 | 4 | 7 | 5.8 | 2 | 5 | 3.3 | 0/5 |
| | 0.3 | 0.7 | 2 | 28 | 8.2 | 3 | 4 | 3.3 | 0/5 |
| 6 | 1 | 0.2 | 3 | 4 | 3.2 | 1 | 2 | 1.3 | 0/5 |
| | 0.5 | 0.4 | 3 | 5 | 3.8 | 1 | 1 | 1 | 0/5 |
| | 0.3 | 0.7 | 4 | 16 | 8.7 | 2 | 3 | 2.3 | 0/5 |
| 7 | 1 | 0.2 | 3 | 4 | 3.5 | 1 | 2 | 1.3 | 0/5 |
| | 0.5 | 0.4 | 3 | 4 | 3.3 | 1 | 3 | 1.7 | 0/5 |
| | 0.3 | 0.7 | 2 | 4 | 3 | 1 | 2 | 1.7 | 1/5 |
| 8 | 1 | 0.2 | 3 | 4 | 3.2 | 1 | 2 | 1.7 | 0/5 |
| | 0.5 | 0.4 | 3 | 5 | 3.7 | 1 | 2 | 1.7 | 0/5 |
| | 0.3 | 0.7 | 4 | 14 | 6.7 | 1 | 4 | 2.3 | 0/5 |
| 9 | 1 | 0.2 | 1 | 1 | 1 | 1 | 1 | 1 | 0/5 |
| | 0.5 | 0.4 | 1 | 1 | 1 | 1 | 1 | 1 | 1/5 |
| | 0.3 | 0.7 | 2 | 3 | 2.3 | 1 | 2 | 1.7 | 0/5 |
| 10 | 1 | 0.2 | 1 | 1 | 1 | 2 | 4 | 3 | 0/5 |
| | 0.5 | 0.4 | 1 | 2 | 1.3 | 2 | 3 | 2.7 | 0/5 |
| | 0.3 | 0.7 | 2 | 3 | 2.7 | 2 | 2 | 2 | 0/5 |
| 11 | 1 | 0.2 | 1 | 1 | 1 | 2 | 2 | 2 | 0/5 |
| | 0.5 | 0.4 | 1 | 2 | 1.2 | 1 | 3 | 2 | 0/5 |
| | 0.3 | 0.7 | 1 | 2 | 1.3 | 2 | 3 | 2.3 | 1/5 |
| 12 | 1 | 0.2 | 1 | 1 | 1 | 3 | 3 | 3 | 0/5 |
| | 0.5 | 0.4 | 1 | 1 | 1 | 3 | 5 | 3.7 | 0/5 |
| | 0.3 | 0.7 | 1 | 1 | 1 | 2 | 4 | 3 | 0/5 |
| 13 | 1 | 0.2 | 4 | 7 | 4.8 | 2 | 4 | 3.3 | 0/5 |
| | 0.5 | 0.4 | 2 | 15 | 5 | 1 | 6 | 3 | 0/5 |
| | 0.3 | 0.7 | 2 | 5 | 3.5 | 1 | 2 | 1.7 | 0/5 |
| 14 | 1 | 0.2 | 8 | 30 | 13.2 | 4 | 6 | 5 | 0/5 |
| | 0.5 | 0.4 | 6 | 15 | 10.2 | 3 | 7 | 5 | 0/5 |
| | 0.3 | 0.7 | 2 | 4 | 2.7 | 2 | 3 | 2.7 | 0/5 |
| 15 | 1 | 0.2 | 1 | 1 | 1 | 1 | 1 | 1 | 0/5 |
| | 0.5 | 0.4 | 1 | 1 | 1 | 1 | 1 | 1 | 1/5 |
| | 0.3 | 0.7 | 1 | 1 | 1 | 1 | 2 | 1.3 | 0/5 |

**Table 2**

| Example No. | Tablet Size cm³/0.2g | Tablet Density g/cm³ | Disintegration Time in Water (sec.) n=6 | | | Disintegration Time in Oral Cavity (sec.) n=3 | | | Disintegration Time with Water Droplets (sec.) n=6 | | | Dropping Strength n=5 (Breakage Rate) |
|---|---|---|---|---|---|---|---|---|---|---|---|---|
| | | | Min. | Max. | Mean | Min. | Max. | Mean | Min. | Max. | Mean | |
| 16 | 1 | 0.2 | 3 | 7 | 4.8 | 3 | 7 | 4.3 | 3 | 3 | 3 | 1/5 |
| | 0.5 | 0.4 | 4 | 25 | 12 | 2 | 4 | 3 | 3 | 3 | 3 | 0/5 |
| | 0.3 | 0.7 | ― | ― | ― | 3 | 4 | 3.3 | 3 | 3 | 3 | 0/5 |
| 17 | 1 | 0.2 | 3 | 26 | 10.5 | 3 | 4 | 3.3 | 2 | 5 | 3.2 | 0/5 |
| | 0.5 | 0.4 | 2 | 15 | 5.5 | 4 | 5 | 4.7 | 4 | 7 | 5 | 0/5 |
| | 0.3 | 0.7 | 7 | 16 | 10.7 | 3 | 6 | 4.3 | 3 | 4 | 3.2 | 0/5 |
| 18 | 1 | 0.2 | 2 | 4 | 3 | 3 | 4 | 3.3 | 6 | 8 | 7 | 0/5 |
| | 0.5 | 0.4 | 2 | 3 | 2.5 | 3 | 4 | 3.3 | 3 | 6 | 4.8 | 0/5 |
| | 0.3 | 0.7 | 3 | 4 | 3.5 | ― | ― | ― | 3 | 4 | 3.2 | 0/5 |
| 19 | 1 | 0.2 | 3 | 14 | 6 | 2 | 4 | 3 | 3 | 5 | 4.5 | 0/5 |
| | 0.5 | 0.4 | 7 | 21 | 13.2 | 4 | 5 | 4.7 | 8 | 18 | 13.2 | 0/5 |
| | 0.3 | 0.7 | ― | ― | ― | 2 | 4 | 3 | 10 | 20 | 16.7 | 0/5 |
| 20 | 1 | 0.2 | 4 | 5 | 4.7 | 3 | 5 | 4 | 10 | 65 | 31.8 | 0/5 |
| | 0.5 | 0.4 | 4 | 6 | 4.8 | 4 | 5 | 4.7 | 8 | 46 | 24.8 | 0/5 |
| | 0.3 | 0.7 | 4 | 40 | 11.5 | 3 | 5 | 4 | 2 | 4 | 2.8 | 0/5 |
| 21 | 1 | 0.2 | 4 | 5 | 4.7 | 4 | 10 | 6.3 | 8 | 42 | 23.5 | 0/5 |
| | 0.5 | 0.4 | 4 | 9 | 6.3 | 3 | 6 | 4 | 5 | 13 | 9.8 | 0/5 |
| | 0.3 | 0.7 | 7 | 10 | 8.7 | 3 | 5 | 4 | 5 | 29 | 11.2 | 0/5 |
| D₁₀ | ― | ― | 52 | 73 | 64.7 | 13 | 17 | 15.3 | 100 | 114 | 108 | 4/5 |
| D₂₀ | ― | ― | 47 | 56 | 51.8 | 13 | 21 | 18 | 80 | 120 | 106.7 | 5/5 |
| G | 0.065* | 1.5 | 11 | 35 | 19 | 22 | 48 | 33 | 210 | 420 | 325 | 5/5 |
| Note: A) D₁₀ and D₂₀ are commercially available orally disintegrating tablets. | | | | | | | | | | | | |
| B) G is a commercially available effervescent tablet (* tablet size: 0.065 cm³/0.1 g) | | | | | | | | | | | | |

As a result of the above tests, it was demonstrated that the orally disintegrating solid preparations of the present invention are disintegrated in a shorter period of time (mean value) than the commercially available orally disintegrating tablets and the solid preparations (tablets) (disclosed in the aforementioned documents known in the art) which have been reported to be quickly dissolved or disintegrated.

Each orally disintegrating solid preparation of examples was disintegrated in the oral cavity almost within 5 seconds, and at the latest within 10 seconds. In water, it was disintegrated almost within 10 seconds and at the latest within 30 seconds. With water droplets, results similar to those in water were obtained although its disintegration time period was prone to be slightly prolonged.

In contrast, the commercially available drugs, D₁₀ and D₂₀ required 13 seconds at the earliest and approximately 20 seconds at the latest for disintegration in the oral cavity, approximately 50 seconds at the earliest and approximately 1 min at the latest in water, and 1.5 min at the earliest and nearly 2 min at the latest with water droplets for disintegration.

Likewise, the effervescent tablet G required 22 seconds at the earliest and 48 seconds at the latest in the oral cavity, 11 seconds at the earliest and 35 seconds at the latest in water, and 3.5 min at the earliest and 7 min at the latest with water droplets for disintegration.

However, most of orally disintegrating solid preparations in the examples were not fractured whereas all of the commercially available tablets tested were fractured except for one tablet of D₁₀ in the dropping strength tests.

### ADVANTAGES OF THE INVENTION

As illustrated in detail above, since the novel orally disintegrating solid preparations of the present invention wherein sugars are utilized as bases are rapidly disintegrated or dissolved within several seconds and at the latest within 10 seconds in the oral cavity, they can be ingested in admixture with liquid foods such as soups and juices; in addition, the preparations allow for extremely easy ingestion due to the sweetness of the sugars. Furthermore, the dropping strength is remarkably improved as compared to the orally disintegrating tablets known in the art.

Therefore, the orally disintegrating solid preparations of the present invention are extremely useful as safe preparations not only for adults but also, especially, for children and the elderly.

Moreover, the novel processes for the formulation according to the present invention are capable of corresponding to large scale production by improving and cogitating the apparatus to dispersively eject melts in the form of filaments, and are anticipated to be utilized for industrial production since not only does the number of the steps decrease compared to the conventional processes in formulation but a large scale equipment for the freeze-drying method is not necessary.

While the present invention has been described specifically in detail with reference to certain embodiments and examples thereof, it would be apparent that it is possible to practice it in other forms. In light of the disclosure, it will be understood that various modifications and variations are within the spirit and scope of the appended claims.

## Claims

1. An orally disintegrating solid pharmaceutical preparation which is prepared by the steps:
dispersively ejecting a melt containing a sugar and one or more pharmaceutically active components under the atmosphere at a temperature equal to or lower than the solidifying point of the sugar, cooling and solidifying the melt to form a flocculent product, and then formulating the flocculent product into a suitable dosage form.

2. The orally disintegrating solid pharmaceutical preparation according to Claim 1, wherein the sugar comprises one or more members selected from the group consisting of monosaccharides, disaccharides and sugar alcohols.

3. The orally disintegrating solid pharmaceutical preparation according to Claim 1 or 2, wherein the sugar is one or more members selected from those having a melting point of from 70 to 250 °C .

4. The orally disintegrating solid pharmaceutical preparation according to any of Claims 1 through 3, wherein the sugar is one or more members selected from sucrose, maltose, lactulose, erythritol, xylitol and sorbitol.

5. The orally disintegrating solid pharmaceutical preparation according to any of Claims 1 through 4, wherein the pharmaceutically active component is one or more drugs selected from drugs for the central nervous system, drugs for cardiovascular organs, drugs for respiratory organs, drugs for gastrointestinal organs, hormone drugs, vitamins, drugs for tumors, drugs for allergy and narcotics.

6. The orally disintegrating solid pharmaceutical preparation according to any of Claims 1 through 5, wherein the pharmaceutically active component is a pediatric drug.

7. The orally disintegrating solid pharmaceutical preparation according to any of Claims 1 through 5, wherein the pharmaceutically active component is a drug for the elderly.

8. A process for producing an orally disintegrating solid pharmaceutical preparation capable of being rapidly dissolved or disintegrated in an oral cavity, which comprises:
(i) adding one or more pharmaceutically active components to a sugar, if necessary, in admixture with an additive, to form a powder mixture,
(ii) heating and melting the powder mixture to give a liquid melt,
(iii) dispersively ejecting the liquid melt from pores or nozzles under an atmosphere at a temperature equal to or lower than the solidifying point of the sugar,
(iv) cooling and solidifying the melt to form a flocculent product, and then formulating the flocculent product into a suitable dosage form.
